# EUROPEAN PATENT SPECIFICATION

(11) **EP 0 771 874 B1**
(45) Date of publication and mention of the grant of the patent: **30.09.1998**
(21) Application number: 96117613.8
(22) Date of filing: 04.11.1996
(51) Int. Cl.: C12N 15/14, C12N 15/85, A01K 67/027

(54) **Transgenic protein production**
Vorbereitung transgenischer Proteine
Production de proteine transgenique

(30) Priority: 03.11.1995 IL 11587395
(43) Date of publication of application: 07.05.1997
(73) Proprietor: STATE OF ISRAEL-MINISTRY OF AGRICULTURE, Bet Dagan 20250 (IL)
(72) Inventor: Barash, Itamar, Rehovot (IL); Shani, Moshe, Macabim, Modiin (IL); Nathan, Margaret, Gaithersburg, Maryland (US); Hurwitz, David R., Boston, Massachussetts (US)
(74) Representative: Benedum, Ulrich Max, Dr.

(56) References cited:
- NUCLEIC ACIDS RESEARCH, vol. 24, no. 4, 1996, pages 602-610, XP000652234 I. BARASH ET AL.: "Elements within the beta-lactoglobulin gene inhibit expression of HSA cDNA and minigenes in transfected cells but rescue their expression in the mammary gland of transgenic mice"
- TRANSGENIC RESEARCH, vol. 1, 1991, pages 3-13, XP000654322 C.B.A. WHITELAW ET AL.: "Targetting expression to the mammary gland: intronic sequences can enhance the efficiency of gene expression in transgenic mice"
- PROCEEDING OF THE NATIONAL ACADEMY OF SCIENCES USA, vol. 85, 1988, page 836-840 XP002033601 R.L. BRINSTER ET AL.: "Introns increase trancriptional efficiency in transgenic mice"

## Description

The present invention relates to a DNA hybrid construct for targeting and efficient expression in the mammary gland of a lactating transgenic mammal, with limited ectopic expression in other tissues. The invention further relates to a transgenic mammal which has a β-lactoglobulin (BLG)/human serum albumin (HSA) cDNA hybrid construct incorporated in its genome. The invention also relates to a method for obtaining said transgenic mammal.

Human serum albumin (HSA) is a globular, non-glycosylated protein (MW 65,000), synthesized by the liver, circulating in the blood stream at levels of 42 g/L. HSA is the most abundant serum protein and involved in a number of essential functions, including sustaining normal blood stream osmolarity, regulating blood pressure, and in transporting fatty acids, amino acids, bile pigments and numerous other small molecules. Clinically, HSA is widely used to replace blood volume in acute phase conditions such as trauma, severe burns or surgically procedures. Currently, the supply of HSA depends on the fractionation of donated human blood. In the moment, the costs of purifying HSA from blood are relatively low since HSA is purified from human blood concurrently with other blood products such as coagulation factors. As these other blood products are increasingly obtained by recombinant DNA technologies, the costs of purifying HSA from blood will increase. The trend of a diminishing supply of donated blood, the rising costs of purifying HSA from blood and the risk of contamination with viruses that cause hepatitis, AIDS and other diseases make an alternative source and approach for the production of large quantities of HSA desirable.

Recombinant DNA technologies have been used increasingly over the past decade for the production of important biological materials such as growth hormone, erythropoeitin, interferons, insulin, plasminogen activator, α-antitrypsin and coagulation factors VIII and IX. A variety of hosts have been utilized for the production of medically important proteins including bacteria, yeast, cultured mammalian cells and animals. In practice, bacteria and yeast often prove unsatisfactory as hosts, because the foreign proteins are often unstable and not processed correctly. In bacteria, recombinant HSA is produced as an insoluble aggregate which requires processing to yield the mature, soluble protein. HSA has also been produced in yeast but at significantly lower levels. In addition, a high proportion was either fragmented, cell associated or insoluble (Sleep et al., Biotechnology **8** (1990), p.42-46; Etcheverry et al., Biotechnology **4** (1986), p.729-730; Quirk et al., Biotech. and Applied Biochem. **11** (1989) p.273-287). In view of these problems, the expression of cloned genes in mammalian tissue culture has been attempted and, in some instances, proven to be a viable strategy. However, batch fermentation of animal cells is expensive and technically demanding.

Transgenic animals have also been proposed as a source for the production of valuable proteins. The production of a transgenic livestock offers a number of potential applications including "Molecular Farming" which is also known as "Gene Farming". The proteins of medical and commercial importance are targeted herein for high level expression and production in the mammary gland and subsequently secreted into the milk. Successful expression of desirable proteins in the milk of transgenics depends on two elements:- (A) the *coding gene sequences* and (B) the *control sequences* used to direct the expression to the mammary gland. The correct juxtaposition of those two elements may affect the ability to achieve tissue specific and high level expression.
(A) The *coding sequences* can be cDNA based, i.e. without intronic sequences, or genomic based. In the latter case, the entire gene can be used or the coding sequences within the cDNA in conjunction with a subset of the native introns (minigenes) can be used to generate the transgenic animal. cDNA based constructs are a more favourable material for genetic manipulations:- (1) They are smaller than genomic fragments and usually integrate more efficiently into the host genome. (2) cDNAs are easier to clone and more readily available than genomic fragments. (3) With the length of the manipulated DNA increases also the chance that a particular restriction site occurs more than once which makes genetic manipulations more difficult. This is especially important for most transgenic techniques, as the DNA to be inserted into the mammalian genome must be cleaved from the prokaryotic vector sequences necessary for cloning and DNA manipulations but which can inhibit the expression. Therefore, the longer the piece of DNA, the more difficult to find restriction enzymes which not cleave internally. Attempts to achieve protein expression with cDNAs rather than with full length genes or minigenes have generally resulted in very low protein yields. The majority of cDNA based constructs for expression in the mammary gland of transgenics are expressed at levels not exceeding a few micrograms per millilitre of milk (Table 1). Brinster et al. (PNAS **85** (1989) p.836-849) have demonstrated that the transcription efficiency of transgenes possessing introns is greater than of cDNA transgenes. This is in line with our earlier findings (WO 93/03164; Shani et al., Transgenic Res. **3** (1994), p.141-151) where we demonstrated the importance of specific HSA gene intron combinations in conjunction with HSA cDNA sequences for high level expression (up to 16 mg/ml) of HSA in the milk of transgenic mice.
(B) *Control sequences* from many milk protein genes have been used to target the expression of desirable genes or cDNAs to the mammary gland of transgenics (see also Table 1). Such sequences were mainly derived from the 5'-regulatory regions of the milk proteins genes. In several cases, sequences of the 3' end were also used. The juxtaposition of these control sequences with the sequences of a desired gene frequently results in uncontrolled, ectopic expression of the gene in tissues other than the mammary gland (see discussion in Barash et al., Transgenic Res. **2** (1993), p.206-276). The sequences of the transgenes are then most likely recognized by regulatory factors present in non-mammary gland tissues or because some regulatory sequences of the milk protein gene are absent in the fusion gene vector construct. So far, no lethal side effect have been associated with the ectopic expression of foreign genes driven by milk protein promoters in transgenic mice. However it is important to better control ectopic expression in order to prevent unforeseen negative consequences of ectopic transgene expression on animal welfare.

From our earlier patents on the production of valuable proteins in the milk of transgenic animals, US-A-5,366,894 (Clark et al.) teaches the use of control sequences from the ovine BLG gene for directing the expression. The DNA of interest was cloned into the *first* untranslated exon of the BLG gene, a strategy which in our experience resulted in a no detectable expression of the HSA cDNA (Shani et al., Transgenic Res. **1** (1992), p.195-208).

WO 94/05796 (Lubon et al.) relates to the expression of Human Protein C in the milk of transgenic pigs. The control sequences were derived from the murine Whey Acidic Protein gene (WAP) and able to direct high level expression (up to 1 mg/ml of foreign protein (i.e. Protein C) to the milk of transgenic pigs from a cDNA based construct (Valender et al., PNAS. USA **89** (1992) p.12003-12007).

The present invention relates to a BLG/HSA cDNA hybrid construct for targeting and expression of HSA in the mammary gland of a transgenic animal, comprising a 5' flanking sequence from a mammalian gene, a sequence coding for HSA and intra-genic BLG sequences for targeting the expression of the cDNA to the mammary gland. More specifically, the present invention provides a construct that can be targeted for efficient expression in the mammary gland of a lactating female transgenic mammal such as mice, rabbits, and farm animals like goats, sheep, pigs and cattle.

The present patent invention demonstrates that insertion of the HSA cDNA into the second BLG exon enabled an efficient expression of the HSA cDNA in the mammary gland. The BLG/HSA hybrid construct is new due to its modified BLG control sequences (compared to those described previously in WO 93/03164 and US-A-5,366,894). Expression levels of up to 0.3 mg HSA/ml milk, driven by the BLG control sequences, are similar in transgenic mice carrying either HSA cDNA or an HSA minigene. Also, the ectopic expression in mice carrying these gene sequences is restricted to skeletal muscle.

The uniqueness of these gene constructs according to the present invention are the presence of intragenic BLG sequences downstream of the transcription initiation site, including exon 1, intron 1 and part of exon 2, as well as intragenic sequences downstream of exon 6 including part of exon 6, intron 6 and exon 7, and BLG 3' untranslated region. The modified BLG control sequences provide a generic vector because they can be used to target and express any cDNA to the mammary gland.

The present invention therefore provides a BLG/DNA construct comprised of a 5'-flanking sequences (e.g. a promoter) and BLG intragenic sequences in conjunction with a desired cDNA, minigene (coding sequences and less than all intron sequences) or gene (coding sequences and all intron sequences) (BLG/DNA hybrid) assembled for the purpose of targeting expression of the product of the encoding "DNA".

The present invention also provides a BLG/DNA hybrid construct wherein the BLG intragenic sequences are comprised of BLG sequences downstream of the BLG translation initiation site including sequences within BLG exon 1, intron 1 and/or exon 2.

The invention further provides for a BLG/DNA hybrid construct wherein the BLG intragenic sequences are comprised of BLG sequences within BLG exon 6, intron 6 and/or exon 7 and/or BLG 3'-untranslated sequences and/or BLG 3'-flanking sequences. The said BLG/DNA hybrid constructs are able in a preferred embodiment of the invention to target expression of the product of the "DNA" in the mammary glands of lactating female transgenic animals.

The present invention also provides a transgenic mammal, selected from, but not restricted to, the group consisting of mice, rabbits, sheep, goats, pigs or cattle generated from constructs (BLG/DNA) as described above. In a preferred embodiment the DNA hybrid construct is comprised of BLG sequences as described above in conjunction with human serum albumin (HSA) cDNA, minigene or gene sequences (BLG/HSA hybrid).

In a preferred embodiment of the present invention, the transgenic mammal is a mouse having incorporated into its genome the BLG/HSA hybrid construct comprised of the HSA cDNA encoding human serum albumin that is produced in the milk of a lactating mouse at levels of approximately 0.3 mg/ml of milk or higher.

The present invention further provides a method for making the said transgenic mammal. This is done e.g. by incorporating the BLG/HSA cDNA hybrid construct into the genome of the mammal. The said invention therefore provides transgenic mammals such as mice, rabbits, sheep, goats, pigs, and cattle, having incorporated into its genome the BLG/HSA cDNA hybrid construct.

In another embodiment of the invention this incorporation is done by microinjecting the construct into the nucleus of fertilized eggs of the mammal which mammal may be selected from the group consisting of, but not restricted to, mice, rabbits, sheep, goats, pigs and cattle.

This method may further comprise testing the said animals for production of HSA in the milk of lactating females and breeding the animals containing the highest level of HSA in the milk to establish a milking herd.

The present invention will now be further described in detail by representative examples 1 of 5. The examples are not intended to be delimiting but to illustrate the scope of the invention.
- **Fig. 1**: illustrates the construction of BLG/HSA hybrid genes - step 1;
- **Fig. 2**: shows the construction of BLG/HSA hybrid genes - step 2;
- **Fig. 3**: shows the BLG regulatory sequences and BLG/HSA constructs used for microinjection;
- **Fig. 4**: shows the used BLG/HSA hybrid constructs:- (A) constructs described in previous patent application containing HSA gene sequences inserted into the first untranslated BLG exon and terminated by the SV40 pA site. - (B) HSA minigenes with various regulatory sequences of the BLG gene and the Major Late Adenovirus promoter.

### Example 1 - Construction of recombinant vectors p838 and p839 for microinjection into fertilized eggs

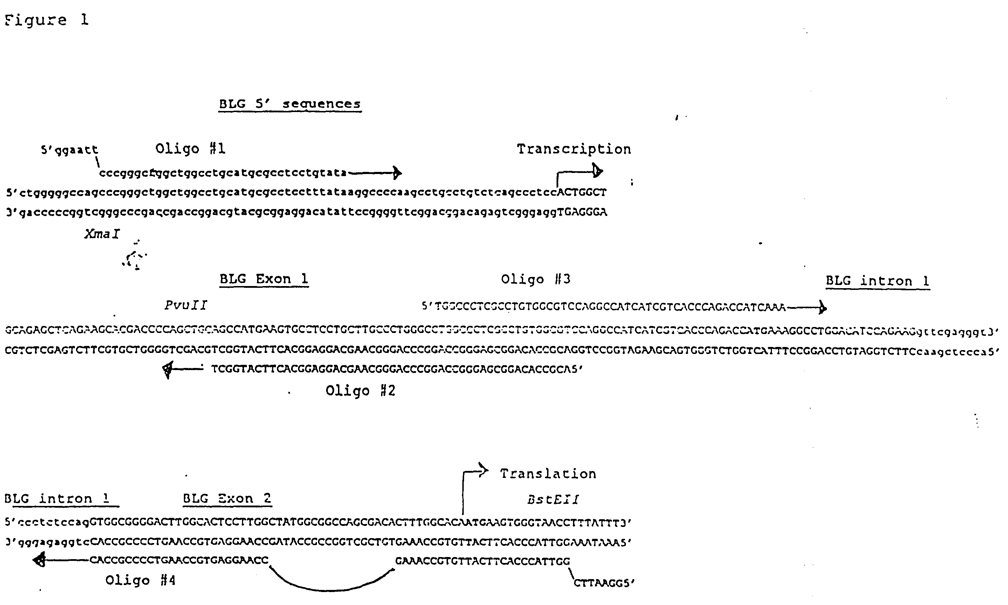

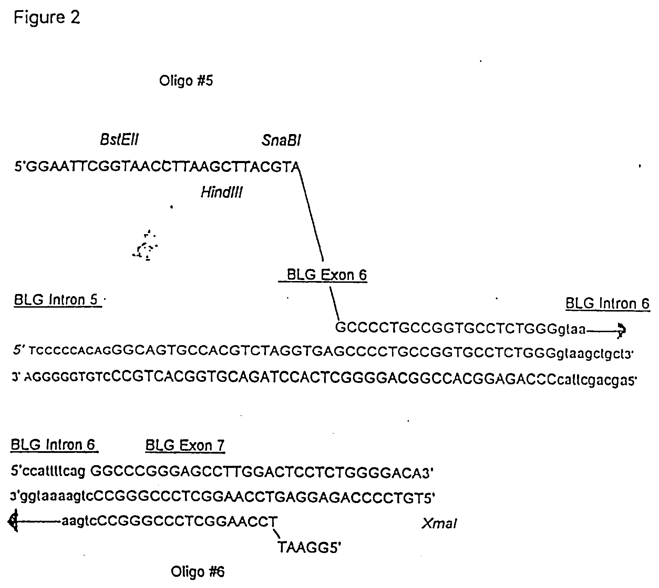

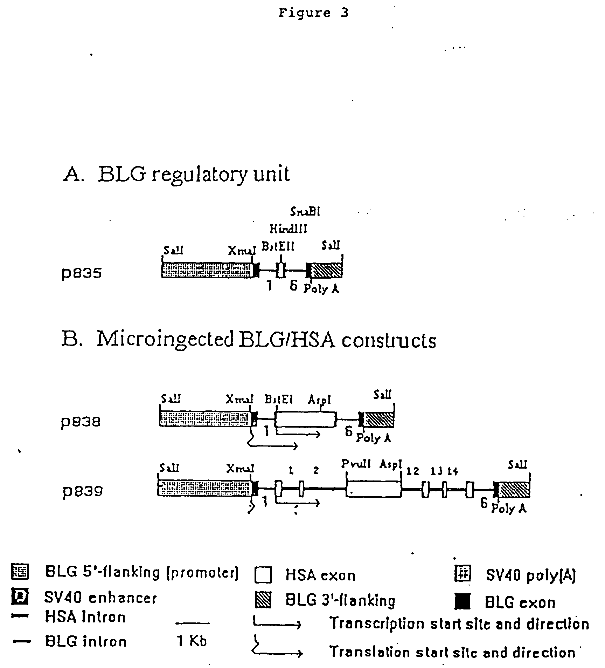

Vectors p838 and p839 were constructed by introducing either the HSA cDNA (p838) or HSA minigene including HSA introns 1,2+12-14 (p839), both of which have the correct Minghetti sequence of amino acid at position #403, into the BstEII/SnaBI site of an earlier construct p835 (described below and in Fig. 3). Construct p835 is a modified p585 BLG transgene vector (see WO 93/03164, Shani et al., Transgenic Res. **1** (1992), p.195-208) wherein the BLG sequences between the first part of BLG exon 2 and the last part of BLG exon 6 (downstream of the BLG TAG termination codon) were deleted. In addition, the two initiating ATG codons in the first BLG exon were changed to ATT and ATC non-initiating sequences as follows: In a first PCR reaction using oligo #1 and oligo #2 (Figure 1) the BLG 5'-sequences and part of BLG exon 1 were amplified. A second PCR reaction, using oligos #3 and #4, amplified an overlapping part of BLG exon 1, the rest of exon 1, BLG intron 1, part of BLG exon 2 and the very 5'-end of the HSA cDNA coding sequences up to its BstEll site. The original 5'-BLG EcoRI clone (p570, described in detail in WO 93/03164) was used as a template for these two PCR reactions because an introduced Sna Bl site within BLG intron 1 of later clones was undesirable in the new vector. Sequences contained within oligos converted two potential BLG ATG translation-initiation codons in BLG exon 1 into non-initiating sequences. A third potential ATG translation-initiation codon in BLG exon 2 was not included in the PCR product or in the new vectors. Overlapping products of the first and second PCR reactions were used as a template for a third PCR reaction using oligos #1 and #4 thereby generating a product possessing the sum of the two original PCR products. A fourth PCR reaction using oligos #5 and #6 (Figure 2) and p585 as template, was used to generate a product with a 5'-BstEII site, a downstream of SnaBI cloning site, BLG exon 6 (downstream of BLG termination codon), BLG intron 6 and exon 7. This product was digested with BstEII and ligated to the product of the third PCR reaction (similarly digested with BstEII). An aliquot of this ligation product was used as template in a fifth PCR reaction which included oligo #1 and oligo #6. The product of this reaction, comprised of all components generated in previous reactions, was digested with XmaI (within BLG 5'-sequences at the 5'-end and within BLG exon 7 and the 3'-end). BLG vector p585 was digested with XmaI, within BLG 5' sequences and within exon 7. The large fragment composed of sequences upstream of XmaI site in BLG 5'-sequences and BLG sequences downstream of the XmaI site in exon 7 was purified. This fragment was ligated to the final PCR product (digested with XmaI) and recombinants with insert in the correct orientation were selected, resulting in construct p835 (Figure 3).

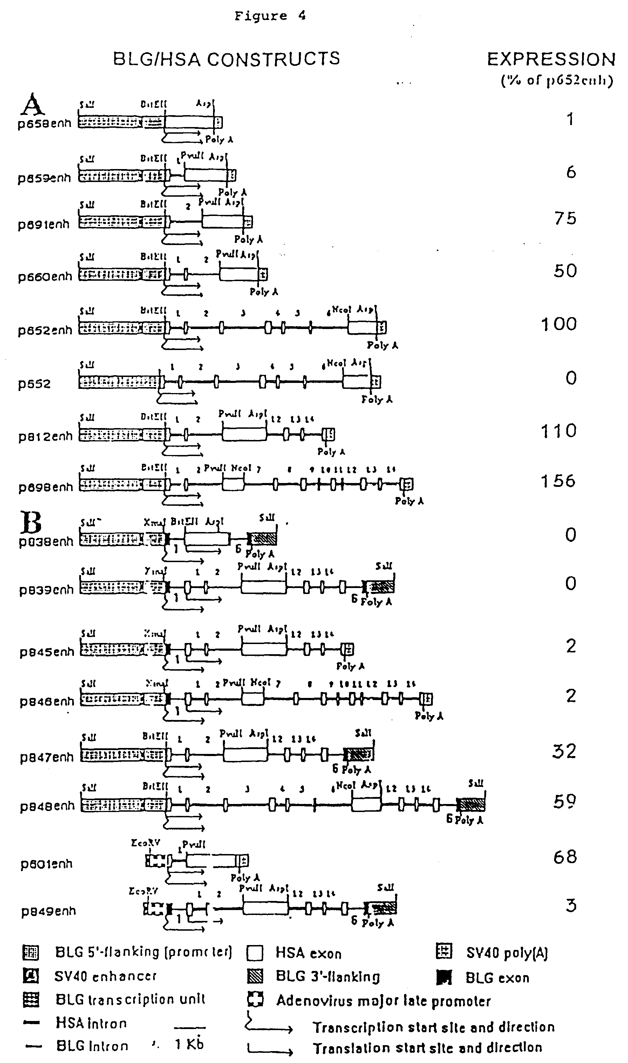

HSA cDNA sequences between the BstEII site in HSA exon 1 and the HindIII site (blunted by filling in with T4 polymerase) downstream of HSA coding sequences, was ligated between the BstEII and SnaBI sites of p835 resulting in the construction of transgenic vector p838 (Fig. 4). Transgenic vector p839 was similarly constructed except that the insert was generated as a BstEII/HindIII (blunted) fragment from construct p826 (described in WO 93/03164) which contained an HSA minigene with HSA intron 1,2 + 12-14 (Fig. 3).

Vector p838 was constructed to support high level expression of HSA cDNA (and as a model for the expression of any other protein from cDNA) in the milk of transgenics. In addition, it restores the tissue specificity and the hormone specific regulated expression in contrast to earlier vectors. Vector p839, with the HSA minigene, was constructed to support high level expression and proper regulation and to serve as a control to compare cDNA with genomic constructs.

### Example 2 - Generation and identification of transgenic mice

### A. Collection of fertilized eggs

Mice used for the collection of fertilized eggs were from the FBV/N inbred line, established at the NIH (Takeo et al., PNAS USA **88** (1991), p.2065-2069) They were obtained from the National Institute of Health Animal Genetic Resource. To induce superovulation, 5-6 week females were injected with 5 i.u. PMSG (intervet), and subsequently (44-48 hours later) with 5 i.u. of Human Chorionic Gonadotropin (HCG) (Sigma Chemical Company). The females were then mated with mature FBV/N males. The following morning, the mated females were identified by the presence of a vaginal plug. The flushing of fertilized eggs from the oviduct, treatment by hyaluronidase and culture condition in M16 or M2 media were performed as described by Hogan, Costantini and Lacy in *"Manipulation of the mouse embryo: A laboratory manual"*, Cold Spring Harbour Laboratory -CSHL (1986).

### B. Preparation of DNA for microinjection

To purify DNA sequences for microinjection, plasmids carrying the BLG/HSA gene were digested with Sall, fragments separated on 1,5% agarose gels, electroeluted and purified on Elutip column (Schleicher & Schuell). DNA was suspended in 10 mM Tris pH 7.5 containing 0.25 mM EDTA at a concentration of 1-3 µg/ml and microinjected into the pronuclei of FBV/N eggs, which were subsequently implanted into the oviduct of CD1 pseudopregnant recipient mice as described in *"Manipulation of the mouse embryo: A laboratory manual"*, CSHL (1986).

### C. Microinjection

Injection pipettes were made from 10 cm long, 1.0 mm outside diameter, thin-walled borosilicate glass capillaries with filament (cat. No. TW100F-4; World Precision Instruments, Inc. 375 Quinniplac Ave. New Haven Conn. 06513, USA). The holding pipettes were prepared from 0.9 cm long, 1.0 mm outside diameter glass capillaries (Ct. No. 105G; Drummond Scientific Co. 500 Pkway., Broomall, PA 19008, USA) as in *"Manipulation of the mouse embryo: A laboratory manual",* CSHL (1986).

Microinjection was carried out in a drop of M2 medium overlaid with silicone oil (Cat. No. 6428-R20; Thomas Scientific, P.O. Box 99, Swedesboro NJ08085-0099, USA), in a glass microscope slide chamber. The chamber was mounted on the microscope (Diaphot, Nikon) equipped with x20 and x40 differential interference contrast (DIC) objectives and x10 eyepieces. 3D hydraulic micromanipulators (Cat. No. MN-188, Nikon) were mounted on the stage of the microscope.

DNA (about 1 µl) was introduced into the injection pipette at the broad side and was carried to the tip by capillary action along the inner filament. The injection capillaries were filed up and mounted onto the micromanipulator via the instrument collar (Cat. No. 070 321; Bunton Instruments Co., Inc. Rockville, MD 20850 USA), which was connected to the hydraulic drive unit (HUB; Bunton Instrument Co. Rockville, MD20850, USA) by a tubing (PE-100 Bunton Instrument, Co. Inc., Rockville MD20850, USA). The holding capillary was similarly mounted. A Pico-Injector (PLI-100m Medical Systems Corp. Greenval, NY) was used to mediate N2 pressure into the microinjection pipette.

Batches of 20-30 pronuclear stage eggs were placed in the injection chamber. The holding and injection pipettes are brought to the chamber. While the holding pipette picked up the egg, the injection pipette was inserted into the pronucleus and about 2 pL of DNA solution injected. When all the eggs in the chamber were injected, they were harvested and cultured for at least 1 hour before implantation.

### D. Embryo transfer

For routine embryo transfer we used the outbred CD1 females mated with vasectomized CD1 males. Between 10-15 microinjected eggs were transferred to each oviduct, essentially as described in *"Manipulation the mouse embryo: A laboratory manual"* CSHL (1986).

### E. Identification of transgenic mice

Transgenic animals were identified by tail biopsies (2 cm) taken 3 weeks after birth. Biopsies wee incubated in 1 ml of 50 mM Tris pH 8.0 containing 0.5% SDS, 0.1 M EDTA and 200 µg proteinase K overnight at 55°C. Genomic DNA was purified from the homogenates by extraction with phenol/chloroform. Approximately 10 µg of DNA from each sample was digested with Bam HI, fractionated on 0.8% agarose and transferred to Gene screen filters (Du Pont). Hybridization was performed at 42°C in 50% formamide, with ³²P-dATP labeled probes made to the insert of plasmid p839, using a random primed DNA labelling kit (Boehringer Mannheim). Filters were washed with 0.2 x SSC containing 1% SDS at 60°C, and exposed to Kodak XAR-5 film at -80°C as described earlier (WO 93/03164).

### Example 3 - Analysis of mammary gland expression

### A. Collection and fractionation of milk

Milk was collected from nursing transgenic mice 10-12 days after parturition. Three hours after mothers were separated from their pups they were injected intraperitonially with 0,3 lU oxytocin (Sigma). Milk was collected 10 min. later by gentle massage of the mammary gland and taken up in a capillary tube. Milk samples were diluted 1:5 in water containing 2 mM PMSF and Aprotinin (Sigma) and defatted by centrifugation. To prepare whey, the caseins were first precipitated by addition of 1 M HCl to pH 4.5. Whey proteins were subsequently precipitated in 10% trichloroacetic acid (TCA), washed with ethanol and acetone and solubilized in SDS-PAGE sample buffer.

### B. Milk protein analysis

Milk proteins were analyzed for the presence of HSA in diluted (1:5) defatted milk collected from lactating G₀ or G₁ females from the indicated transgenic strains. To confirm that the proteins had the correct molecular weight, HSA was analyzed on 7.5% SDS-PAGE. Final quantification of HSA in the milk was performed by immuno dot blot of 1 µl milk samples with HSA standards ranging from 0.002 to 1 µg/lane. Signals were generated on Kodak XAR-5 film after incubation of the filters with ¹²⁵I-anti HSA monoclonal antibodies as described in WO 93/03164.

### C. Expression of HSA RNA in different tissues of transgenic mice.

In order to examine the tissue specificity of HSA expression, total RNA was isolated from various tissues of transgenic female mice on day 10-12 of lactation. Total RNA was extracted by the LiCl/urea procedure, RNA (10-15 µg) was fractionated on MOPS/formaldehyde agarose gels and blotted on Nitran nylon filter and hybridized to a ³²P-labeled HSA cDNA probe. The HSA probe cross-hybridized to the endogenous mouse serum albumin mRNA in liver. However, this endogenous RNA had a higher mobility on agarose gels due to its shorter size as shown in our previous patent application. Thus, the different characters of the HSA and the endogenous albumin enabled us to use the liver RNA as a control for our hybridization analysis and to differentiate between the expression of HSA RNA and endogenous mouse albumin RNA in the kidney.

### Example 4 - Transient expression of recombinant vectors in vitro

### A. Construction of recombinant vectors p838enh, p839enh, p845enh, p846enh, p847enh, p848enh, p849enh. and p601enh for in vitro transient expression.

*In vitro* transient expression vectors were generated from vector p838 and p839 by fragment switching with an earlier transient vector [designated p652(1-6)*enh, Hurwitz et al. Transgenic Res. **3** (1994), p.365-375 and WO 93/03164], resulting in the introduction of an SV40 enhancer about 900 bp upstream of the BLG transcription-initiation codon. Those vectors were designated p838enh and p839enh, respectively (Fig. 4). Vectors p845enh and p846enh possess the BLG 5'-sequences used in p838enh and p839enh (including BLG exon 1/intron 1/exon 2), HSA minigene including HSA introns 1,2 + 12-14 or 1,2 + 7-14, respectively, and an SV40 poly(A) site which replaced the BLG 3'-sequences downstream of the Ncol site (BLG exon6/intron 6/exon 7, (Hurwitz et al. Transgenic Res. **3** (1994) p365-375) A third set of vectors was constructed that maintained the BLG 3'-sequences (exon 1/intron 1/exon 2) with the normal BLG promoter/exon 1 sequences used in all earlier vectors (Shani et al. Transgenic Res. **1** (1992) 195-208; Hurwitz et al. **3** (1994) p.365-375) Vector p847 has an HSA minigene with HSA introns 1,2 + 12-14 and vector p848 has an HSA minigene with HSA introns 1-6 + 12-14. Vector p849enh completely replaced the BLG promoter and SV40 enhancer from p839enh with an adenovirus major late promotor and SV40 enhancer (Hurwitz et al., 1987). The promoter itself is 402 bp. It is immediately followed by the continuous adenovirus major late leader sequences (L1, L2 and most of L3) of 173 bp. The SV40 enhancer (179 bp) is positioned at the very 5'-end of the major late promoter at an EcoRV site. Vector p849enh still maintains BLG exon 1/intron 1/exon 2/ and HSA minigene with introns 1,2 + 12-14/BLG exon 6 intron 6 exon 7 as the transcription unit. Vector p601enh contains the SV40 40 enhancer in front of the Adenovirus Major Late Promoter and HSA minigene containing HSA intron 1 in its native position. This vector is terminated by the SV40 polyadenylation site.

### B. In vitro (tissue culture) analysis of BLG/HSA vectors

In order to determine whether the BLG/HSA vectors introduced into transgenic animals have the potential to support the expression of HSA in the milk of such animals, their ability to drive HSA expression was first tested in tissue culture cells. The natural *in vivo* regulation of expression of milk proteins under the control of their native promoters (e.g. BLG) is complex and requires the influence of hormones and specific cell-cell interactions. The BLG 5'-flanking promoter sequences are not usually active in tissue culture cells. Furthermore, tissue culture systems which precisely mimic the natural *in vivo* conditions do not exist. Therefore, to test the activity of these (i.e. BLG) sequences an SV 40 enhancer was introduced within the promoter. This allowed testing of the levels of expression of HSA in tissue culture cells from BLG/HSA constructs which differ in their HSA make-up (cDNA, minigene, gene). In order to maintain the same genetic background in these *in vitro* analysis constructs, p652 containing the SV40 enhancer was used as the source for all of them as described in our previous patent. The detailed methodology of introducing the SV40 enhancer approximately 900 bp upstream of the BLG transcription initiation site is also described in WO 93/03164.

The *in vitro* tissue culture expression was accomplished by transient transfection. Tissue culture mammalian cell line COS-7 cells were split equally into 100 mm tissue culture dishes in DMEM medium plus 10% fetal calf serum (FCS) so they were approximately 50% confluent (approximately 5x10⁶ cells). They were incubated overnight at 37°C in a CO₂ incubator. The next morning, the medium was replaced with 5 ml of fresh medium and cells were incubated for 1-2 hours. They were then transfected with BLG/HSA constructs (which included the SV40 enhancer) using the calcium phosphate technique (reagents supplied by the 5'-3' Inc.) by the supplier's protocol. In each experiment the total amount of the largest construct (kb) transfected into cells was 25 µg. To transfect equal molar amounts of smaller constructs, the amount of each of these constructs were reduced proportionally to their size difference to the largest construct and the total amount of DNA for the each construct was brought up to 25 µg using high molecular weight (HMW) salmon sperm (ss) DNA. Following transfection for 4-5 hours, cells were glycerol shocked (3 ml, 2 min) and washed according to the supplier's protocol. Subsequently, cells were incubated in 10 or 15 ml of DMEM medium plus 10% FCS for 3 days.

To detect expression and secretion of HSA, transfected cells were starved for amino acids cysteine (Cys) and methionine (Met) by first washing with and then incubating in DMEM medium (plus glutamine), lacking Cys and Met, plus 5% dialyzed FCS (dFCS) for 1-3 hours. Following the removal of medium, cells (and *de novo* synthesized proteins) were metabolically labeled in 3 ml DMEM (plus glutamine, without Cys or Met, plus 10% dialyzed FCS) containing ³⁵S-Cys and ³⁵S-Met (Expre³⁵S³⁵S³⁵SS-protein labelling mix; New England Nuclear, Inc.) at approximately 200 µCi/ml for 4-5 hours.

After metabolic labelling, the media supernatant were harvested from dishes and centrifuged to remove any contaminating cells. Metabolically labeled HSA expressed and secreted into supernatant was detected by immunoprecipitation using rabbit anti-HSA antibodies (DAKO - immunoglobulin cat. #A001). Prior to immunoprecipitation, supernatants were precleaned with 200 µl of 50% slurry of protein A-sepharose beads in immunoprecipitation (IPP) buffer (20 mM Tris, pH 8.0, 150 mM NaCl, 1% NP-40, 0.1% SDS, 2 µg/ml Aprotinin) at 4°C for 30-60 min with rocking. Cleared supernatants were recovered by centrifugation and treated with rabbit anti-HSA IgG prebound to protein A-Sepharose beads, at 4°C for 3-4 hours. Beads were washed 6 times with cold IPP buffer, resuspended in 2x SDS-PAGE Laemmli sample buffer, heated to 95°C for 5 min and run on 8% SDS-PAGE gels. Following electrophoresis, gels were fixed (10% acetic acid, 25% isopropanol), treated with the fluorographic reagent Amplify (Amersham, Inc.), dried onto Whatmann 3MM paper and used to expose x-ray films. Developed films (autoradiographs) allowed visualization of the relative levels of expression and secretion of metabolically labeled HSA from each of the tissue culture transient assay plates as supported by each of the analyzed BLG/HSA constructs.

### C. Levels of HSA synthesis driven by the in vitro transfected constructs.

Cos 7 cells were transiently transfected with the indicated BLG/HSA vectors (Fig. 4) and synthesis of the HSA was determined by immunoprecipitation assay as described. The signals on the film indicating the rate of the *de novo* HSA synthesis driven by each of the constructs were quantified by densitometry.

HSA expression in cells transfected with vector p652 enh served as an internal reference to evaluate HSA expression in the various assays and all quantified signals were related to its expression (which is taken as 100%). Transfection of p652 lacking the SV40 enhancer served as negative control and as expected it did not support any HSA expression (0%).

Comparable analysis of expression driven by BLG/HSA vectors displayed in panel A was already shown in WO 93/03164.

*Panel A*: All BLG/HSA constructs shown in panel A contain the BLG 5' regulatory sequences up to the untranslated part of BLG exon 1 and the SV40 PA site at their 3' end. In the present patent application, these vectors serve as controls and reference point for the reliable analysis of the expression of the newly generated vectors - demonstrated in panel B. The general purpose of the comparison between panels A and B was to determine the role of the extended BLG 5'-and 3#-sequences on expression *in vitro*.

As seen in panel A, HSA cDNA (represented by vector p658enh) was expressed at very low levels in cultured Cos cells and addition of HSA intronic sequences in their native positions dramatically increased expression. Addition of the HSA intron 1 (p659enh) and especially HSA intron 2 (p691 enh) in their native positions increased HSA expression up to 75% of that seen with our positive control p652enh.

The lower expression of construct p660enh containing HSA intron 1 and 2 compared to p651enh containing only the 1st HSA intron may suggest negative regulatory elements in HSA intron 2.

Vector p812enh contains HSA introns 1,2 + 12-14. It was expressed at 110% of the level of expression seen with p652enh. Vector p698enh containing the first two and the last 8 (1 + 1 + 7-14) HSA introns in their native positions was expressed at a high level of 156% of that seen with vector p652enh. It indicates that control sequences from both the 5' and the 3' end of the gene acted together for high level expression.

*New BLG/HSA vectors in panel B*: Vector p838enh contains an HSA cDNA as in vector p658enh. The difference between those two vectors is that in p838enh the, HSA cDNA is inserted into BLG exon 2 and followed by the BLG 3' end (BLG exon 6/intron6/exon7) as described in the introduction. No *in vitro* expression was supported by this vector (panel B).

Surprisingly, *in vitro* expression could also not be seen with vector p839enh containing HSA introns 1,2 and 12-14. This result was highly unexpected since in contrast to the analogous vector p812enh containing the same HSA gene sequences, these HSA intronic sequences could not induce HSA expression.

The conclusion from this comparison is that sequences within BLG exon 1/intron 1/exon 2 and/or sequences at the 3' end of the BLG gene completely abrogate the *in vitro* BLG/HSA expression.

To systematically analyze the role of each part of the BLG control sequences in the dramatic decrease in BLG/HSA expression, a series of additional vectors were constructed and transfected to Cos cells.
a) Replacement of BLG 3' sequences downstream of exon 6 with the SV40 pA sequences. BLG sequences downstream of exon 6 were replaced with previously used SV40 sequences. The resulting conducts p845enh and p846enh were analogues of p812enh and p698enh (panel A), respectively, except for the 3' end of the gene.
   While vectors p612enh and p698enh were highly expressed in cultured Cos cells, at 110% and 156% of the expression seen with p652enh, their analogues p845enh and p846enh conferred a very low level of HSA expression (2% of p652enh expression). Therefore, these findings could not be due to specific HSA intronic exonic combination.
   That the 3' SV40 pA site could not rescue HSA expression in these vectors indicate that BLG sequences at the 3' end of the gene are not, by themselves, involved in the abrogation of HSA expression in the new BLG/HSA vectors.
b) Replacement of the downstream extended BLG 5'-sequences with BLG sequences terminating within exon 1. Two HSA minigenes-(intron 1,2 + 12-14 and 1-6 + 12-14) were inserted into the first BLG untranslated exon as in panel A. The resulting constructs p847enh and p848enh, respectively, still contain the BLG sequences downstream of exon 6.

Vector p847enh, an analog of p812enh, conferred 32% of the HSA expression seen with vector p652enh whereas vector p848enh, comprised of additional HSA introns from the 1 st half of the gene, conferred 59% expression of vector p652enh.

The conclusion from these experiments are: (1) BLG control sequences lacking extended BLG exon 1/intron 1/exon 2 sequences direct expression of HSA in Cos cells in an HSA intron dependent manner. (2) The SV40 pA is more potent than BLG 3' sequences for HSA expression.

### D. The role of BLG sequences (exon 1/intron 1/exon 2 and 3' end) in regulating HSA expression driven by a different promoter, the Adenovirus Major Late Promoter.

The Adenovirus Major Late Promoter in conjunction with the SV40 enhancer conferred higher HSA expression compared to the BLG promoter (p601enh vs. p659enh). Still, this promoter could not rescue expression of the HSA minigene, p849enh (containing introns 1,2 + 12-14) in the presence of BLG exon 1/intron 1/exon 2 and the 3'-BLG exon 6/intron 6/exon 6 sequence.

The conclusion from this experiment is that the abrogation of HSA expression by BLG sequences in exon 1/intron 1/exon 2 is not specific to the BLG promoter.

### Example 5 - Mammary-specific and ectopic HSA RNA expression. HSA secretion into the milk by strains carrying constructs p838 and p839.

Table 2 summarizes the level of HSA secretion into milk and the accumulation of HSA RNA in transgenic strains carrying constructs p838 and p839.

### A. HSA secretion to milk and expression in the mammary gland.

Five out of nine transgenic mice carrying vector p838 (HSA cDNA lacking HSA introns secrete detectable levels of HSA into the milk. In six of them HSA mRNA was detected in the mammary gland. This difference is probably due to the different sensitivity of the assays. The level of HSA detected in the milk did not exceed 0.3 mg/ml. This level was detected in the milk of three independent transgenic strains (#130, #155 and #147). Lower levels of HSA, up to three orders of magnitude were detected in the other two strains.

The milk of nine out of eleven mice carrying construct p839 contain detectable levels of HSA. However, the level did not exceed that seen in mice carrying cDNA based vector p838 and reached a comparable level of 0.3 mg/ml.

HSA mRNA was detected in the mammary gland of six out of eleven transgenic strains analyzed. Comparable variation in HSA protein levels secreted to milk and in HSA mRNA detected in the mammary gland was observed among transgenics carrying the intronless construct p838 and the HSA minigene p839.

### B. HSA RNA expression in mouse tissues.

The HSA cDNA probe hybridized to both the HSA and the endogenous mouse albumin mRNAs. However, HSA mRNA could be easily distinguished from the endogenous mouse albumin due to its lower mobility on 0.8% agarose gels. To test how strictly the BLG elements control HSA expression in the mammary gland in terms of tissue specificity, we analyzed endogenous and ectopic expression of HSA mRNA in tissues of transgenic mice carrying constructs p838 and p839. Skeletal muscle, kidney, salivary gland and brain were chosen for analysis since they posed a favourable target for HSA ectopic expression in transgenics carrying HSA minigenes fused to a BLG promoter lacking exon 1/intron 1/exon 2 (Shani et al. Transgenic Res. **1** (1992) p.195-208; Barash et al. Transgenic Res. **2** (1993) p.266-276). The only tissue expressing HSA RNA other than the mammary gland was skeletal muscle. However, correlation could not be drawn between the level of expression in the muscle and in the mammary gland since several strains that express low levels of HSA in the mammary gland (i.e. #145, #137, #149) displayed higher level of HSA mRNA in the muscle while most strains expressing high level of HSA MRNA in the mammary gland express lower ectopic levels of HSA mRNA in the skeletal muscle. Interestingly, the presence of HSA intronic sequences did not alter the sites or the pattern of this ectopic expression in transgenic mice carrying p839 compared to their HSA cDNA carrying counterparts. The endogenous mouse albumin was expressed and detected in the kidney of about 17% of the tested mice.

### C. BLG/HSA transgenes do not confer copy number dependent expression in the mammary gland of transgenic mice.

Many transgenes are strongly influenced by their site of integration in the host chromosome due to the nature of the host flanking sequences as well as the number and arrangement of transgene copies in the array. To determine whether downstream BLG sequences included in the new vectors conferred position independent behaviour of the BLG/HSA constructs, we analyzed the level of HSA secreted from the mammary gland of independent strains of transgenic mice carrying constructs p838 and p839 with respect to the relative copy number of BLG/HSA construct integrated. The relative copy number was calculated as the ratio of the densitometric value of the BLG/HSA signal obtained by "Southern" analysis vs. the value for the β-actin gene signal. β-actin is a single copy gene and represents an internal control reference value for the amount of genomic DNA loaded on the gel. As can be seen in Table 3, no significant correlation could be drawn between the values of HSA excreted into the milk and the relative copy number of the BLG/HSA gene integrated.

**Table 3.**

| Correlation between HSA secretion into milk and BLG/HSA copy number | | |
|---|---|---|
| Construct | n | r value (HSA/actin) |
| p838 | 8 | -0.4 |
| p839 | 6 | -0.33 |
| | 16 | -0.3 |
| The level of HSA secreted into the milk was analyzed by dot blot. The BLG/HSA copy number was determined by Southern analysis of BamHl cut genomic DNA from transgenic mice of different strains which was hybridized once with labeled HSA cDNA and then reprobed with labeled α-actin cDNA. | | |

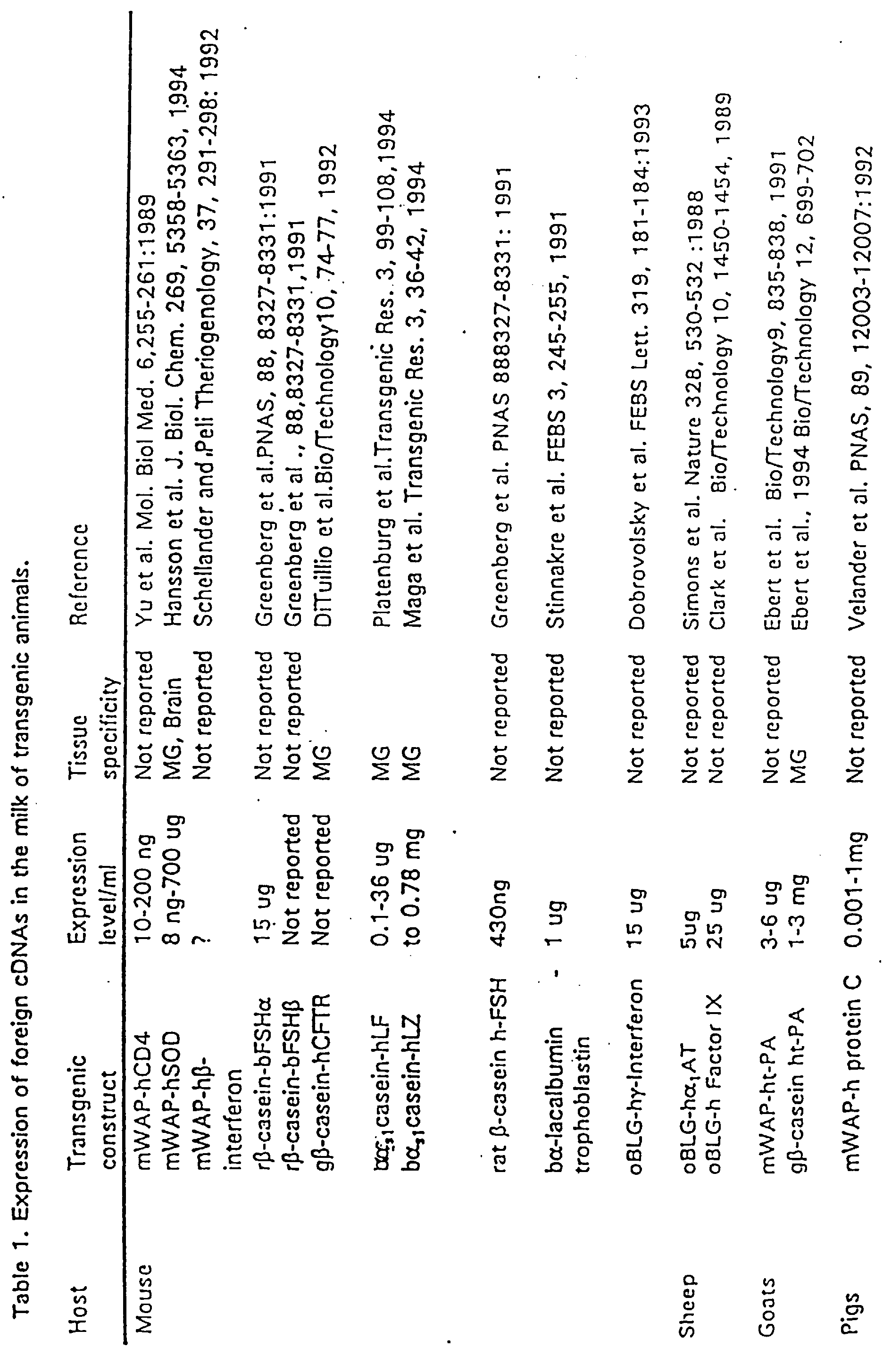

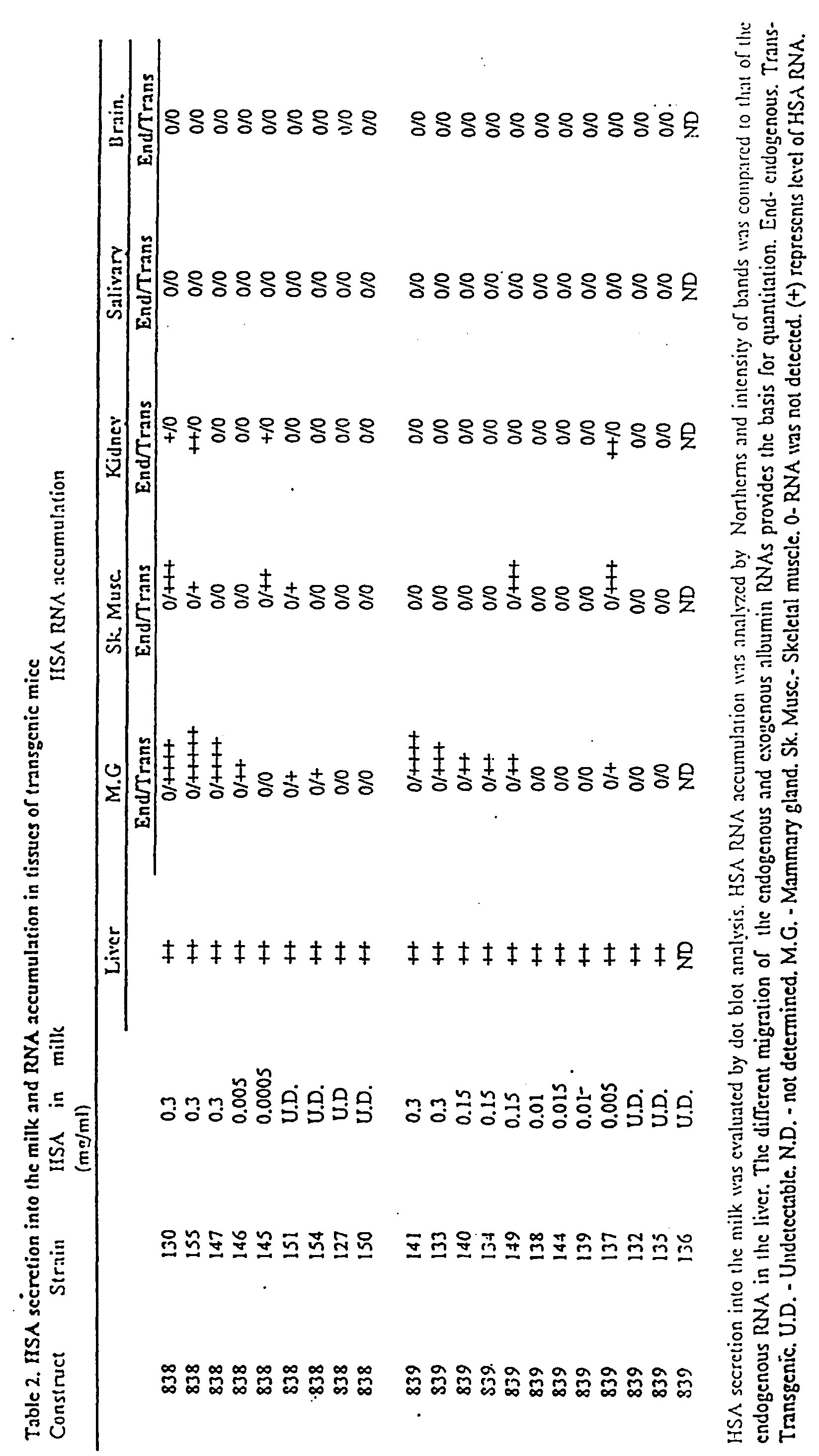

## Claims

1. A β-lactoglobulin (BLG) hybrid construct comprised of BLG 5'-flanking sequences and BLG *intragenic* sequences in conjunction with a desired cDNA or minigene or gene for targeting expression of the product of the encoding DNA.

2. The BLG/DNA hybrid construct according to claim 1 wherein the BLG intragenic sequences are comprised of BLG sequences downstream of the BLG translation initiation site including sequences within BLG exon 1, intron 1 and/or exon 2.

3. The BLG/DNA hybrid construct according to claim 1 wherein the BLG intragenic sequences are comprised of BLG sequences within BLG exon 6, intron 6 and/or exon 7 and/or BLG 3'-untranslated sequences and/or BLG 3'-flanking sequences.

4. The BLG/DNA hybrid construct according to any claim 1 to 3 in conjunction with human serum albumin (HSA) cDNA, minigene or gene sequences (BLG/HSA hybrid).

5. The BLG/DNA hybrid constructs according to any claim 1 to 4, wherein the said construct targets expression of the product of the "DNA" in the mammary gland of lactating female transgenic animals.

6. A transgenic mammal generated from a BLG/DNA hybrid construct as claimed in any claim 1 to 5.

7. The transgenic mammal as claimed in claim 6 selected from the group consisting of mice, rabbits, sheep, goats, pigs, or cattle

8. The transgenic mammal according to claim 7 wherein the mammal is a mouse having incorporated into its genome the BLG/HSA hybrid construct comprised of the HSA cDNA encoding human serum albumin that is produced in the milk of a lactating mouse at levels of approximately 0.3 mg/ml of milk or higher.

9. A method for making a transgenic mammal by incorporating into its genome the BLG/DNA hybrid construct as defined in any claim 1 to 5.

10. The method for making a transgenic mammal by incorporating into its genome the BLG/HSA hybrid construct as defined in claims 4 or 5.

11. The method according to claim 9 or 10 comprising micro-injecting the BLG/DNA construct into an embryo of the mammal.

12. The method according to claim 10 further comprising testing the animal for production of human serum albumin in the milk of lactating females and breeding the animals containing the highest levels of human serum albumin in the milk.

13. A process of obtaining human serum albumin comprising breeding of one or more transgenic animals as defined in any claim 6 to 8 or obtained by a method as claimed in any claim 9 to 12 and isolation of human serum albumin from the milk of lactating female transgenes.

## Patentansprüche

1. β-Lactoglobulin(BLG)-Hybridkonstrukt, beinhaltend 5'-flankierende BLG-Sequenzen und intragene BLG-Sequenzen zusammen mit einer gewollten cDNA, einem Minigen oder einem Gen, für ein zielgerichtetes Lenken der Expression des Produkts, das die DNA kodiert.

2. BLG/DNA-Hybridkonstrukt nach Anspruch 1, wobei die intragenen BLG-Sequenzen BLG-Sequenzen beinhalten, welche stromabwärts der Initiationsstelle für die Translation des BLGs liegen und Sequenzen umfassen, die im ersten Exon, im ersten Intron und/oder im zweiten Exon des BLGs liegen.

3. BLG/DNA-Hybridkonstrukt nach Anspruch 1, wobei die intragenen Sequenzen des BLGs gebildet werden von BLG-Sequenzen, die im sechsten Exon, im sechsten Intron und/oder im siebten Exon des BLGs liegen, und/oder von 3'-untranslatierten Sequenzen des BLGs und/oder von 3'-flankierenden Sequenzen des BLGs.

4. BLG/DNA-Hybridkonstrukt nach irgendeinem Anspruch 1 bis 3 in Verbindung mit einer cDNA, einem Minigen oder einem Gen für Human-Serumalbumin(HSA) (BLG/HSA-Hybrid).

5. BLG/DNA-Hybridkonstrukte nach irgendeinem Anspruch 1 bis 4, wobei das Konstrukt die Expression des "DNA-Produkts" in die Milchdrüse eines laktierenden weiblichen transgenen Tieres lenkt.

6. Transgenes Säugetier, erschaffen aus einem BLG/DNA-Hybridkonstrukt nach irgendeinem Anspruch 1 bis 5.

7. Transgenes Säugetier nach Anspruch 6, ausgewählt aus der Gruppe Mäuse, Kaninchen, Schaf, Ziege, Schwein oder Rind.

8. Transgenes Säugetier nach Anspruch 7, wobei das Säugetier eine Maus ist, in deren Genom ein BLG/HSA-Hybridkonstrukt eingebaut ist, umfassend eine HSA-cDNA, die Human-Serumalbumin kodiert, das dann in der Milch einer laktierenden Maus vorkommt in einer Konzentration von etwa 0,3 Milligramm pro Milliliter Milch oder mehr.

9. Verfahren zur Erschaffung eines transgenen Säugetiers, indem in dessen Genom ein BLG/DNA-Hybridkonstrukt nach irgendeinem Anspruch 1 bis 5 eingebaut wird.

10. Verfahren zur Erschaffung eines transgenen Säugetiers, indem in dessen Genom das BLG/HSA-Hybridkonstrukt nach Anspruch 4 oder 5 eingebaut wird.

11. Verfahren nach Anspruch 9 oder 10, umfassend das Mikroinjizieren des BLG/DNA-Konstrukts in einen Embryo des Säugetiers.

12. Verfahren nach Anspruch 10, zudem umfassend das Testen des Tieres auf die Erzeugung von Human-Serumalbumin in der Milch eines laktierenden Weibchens und Züchten des Tieres, das den höchsten Spiegel an Human-Serumalbumin in der Milch hat.

13. Verfahren zur Gewinnung von Human-Serumalbumin, umfassend das Züchten ein oder mehrerer transgener Tiere, nach einem der Ansprüche 6 bis 8 oder Erhalten mit einem Verfahren nach einem der Ansprüche 9 bis 12, und Isolieren von Human-Serumalbumin aus der Milch eines laktierenden weiblichen transgenen Tieres.

## Revendications

1. Construction d'hybride de β-lactoglobuline (BLG) composée de séquences 5'-flanquantes de BLG et de séquences *intragéniques* de BLG en conjonction avec un ADNc ou un minigène ou un gène désiré pour cibler l'expression du produit de l'ADN codant.

2. Construction d'hybride BLG/ADN suivant la revendication 1, dans laquelle les séquences intragéniques de BLG sont composées de séquences de BLG en aval du site d'initiation de traduction de BLG incluant des séquences à l'intérieur de l'exon 1, de l'intron 1 et/ou de l'exon 2 de BLG.

3. Construction d'hybride BLG/ADN suivant la revendication 1, dans laquelle les séquences intragéniques de BLG sont composées de séquences de BLG à l'intérieur de l'exon 6, de l'intron 6 et/ou de l'exon 7 de BLG et/ou de séquences non traduites en 3' de BLG et/ou de séquences 3'-flanquantes de BLG.

4. Construction d'hybride BLG/ADN suivant l'une quelconque des revendications 1 à 3, en association avec des séquences d'ADNc, de minigène ou de gène de sérum-albumine humaine (HSA) (hybride BLG/HSA).

5. Constructions d'hybride BLG/ADN suivant l'une quelconque des revendications 1 à 4, dans lesquelles cette construction cible l'expression du produit de 1"'ADN" dans la glande mammaire d'animaux transgéniques femelles en lactation.

6. Mammifère transgénique généré à partir d'une construction d'hybride BLG/ADN suivant l'une quelconque des revendications 1 à 5.

7. Mammifère transgénique suivant la revendication 6, choisi dans le groupe consistant en souris, lapins, moutons, chèvres, porcs ou bétail.

8. Mammifère transgénique suivant la revendication 7, dans lequel le mammifère est une souris dans le génome de laquelle est incorporée la construction d'hybride BLG/HSA composée de l'ADNc de HSA codant la sérum-albumine humaine qui est produite dans le lait d'une souris en lactation à des niveaux d'environ 0,3 mg/ml de lait ou plus.

9. Procédé pour fabriquer un mammifère transgénique par incorporation dans son génome de la construction d'hybride BLG/ADN suivant l'une quelconque des revendications 1 à 5.

10. Procédé pour fabriquer un mammifère transgénique par incorporation dans son génome de la construction d'hybride BLG/HSA suivant l'une quelconque des revendications 4 ou 5.

11. Procédé suivant les revendications 9 ou 10 comprenant une microinjection de la construction BLG/ADN dans un embryon du mammifère.

12. Procédé suivant la revendication 10, comprenant de plus le test de l'animal pour évaluer la production de sérum-albumine humaine dans le lait de femelles en lactation et l'élevage des animaux contenant les plus forts taux de sérum-albumine humaine dans le lait.

13. Procédé pour obtenir de la sérum-albumine humaine comprenant l'élevage d'un ou de plusieurs animaux transgéniques suivant l'une quelconque des revendications 6 à 8 ou obtenus par un procédé suivant l'une quelconque des revendications 9 à 12 et l'isolement de la sérum-albumine humaine à partir du lait de transgènes femelles en lactation.
